# EUROPEAN PATENT SPECIFICATION

(11) **EP 2 402 759 B1**
(45) Date of publication and mention of the grant of the patent: **26.02.2014**
(21) Application number: 11169653.0
(22) Date of filing: 10.06.2011
(51) Int. Cl.: G01N 33/58

(54) **Testing strip for detecting a fluidic sample**
Teststreifen zur Detektion einer Fluidprobe
Bandelette réactive pour détecter un échantillon liquide

(30) Priority: 29.06.2010 TW 099121197
(43) Date of publication of application: 04.01.2012
(73) Proprietor: Actherm Inc., Hsinchu 30078 (TW)
(72) Inventor: Hsieh, Wen-Pin, 352 Miaoli County (TW); Wang, Cheng-Hsien, 300 Hsinchu City (TW)
(74) Representative: Lang, Christian

(56) References cited:
- WO-A1-2004/113901
- US-A1- 2006 175 199

## Description

### BACKGROUND OF THE INVENTION

### 1. Technical Field

The present invention relates to a testing strip for detecting a fluidic sample, and more particularly, to a testing strip for detecting a fluidic sample that possesses specific design for biological sampling region.

### 2. Description of Related Art

Analytical strips are conventionally used in biochemical tests and immunological tests. A typical analytical strip has a substrate or a base formed with channels or microfluidic channels and processed with hydrophobic and/or hydrophilic surface treatments. Since the channels are bordered by non-absorbent material, and fluidic samples to be tested usually contain viscous compositions, for example, proteins or carbohydrates, a fluidic sample flowing in the channels tends to adhere to surfaces of the channels and cannot be fully reacted. Consequently, the fluidic sample is wasted, if not leading to errors of test results.

In addition, during the delivery of the fluidic samples in the conventional analytical strips that are provided with channels, air bubbles of various sizes tend to be generated in, or entrained into, fluidic samples to be tested after the samples are introduced into the channels. These bubbles, when causing channel blockage, may result in test errors or even test failure.

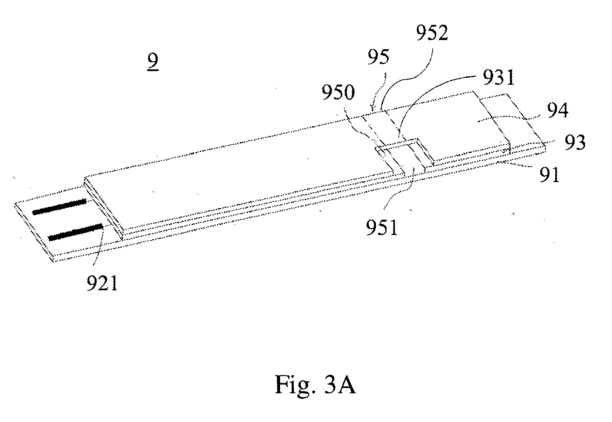

For example, as shown in FIG. 3A, which is a conventional electrochemical-based analytical strip 9 which comprises a substrate 91, a plurality of electrodes 921, an insulating layer 93 and a cover 94. The substrate 91, the plurality of electrodes 921, the insulating layer 93 and the cover 94 are serially stacked. In addition, a transverse groove 931 is interposed within the insulating layer 93, and the transverse groove 931 is parallel to the short axis of the electrochemical-based analytical strip 9. Therefore, after assembled, a channel 95 is defined within a region enclosed by the transverse groove 931, the substrate 91 and the cover 94. The channel 95 is parallel to the short axis of the electrochemical-based analytical strip 9 and disposes a reaction region 950 in the middle section thereof.

Such electrochemical-based analytical strip 9 respectively disposes an opening 951 and an opening 952 at its both lateral sides. Thereby, either the opening 951 or the opening 952 is capable of being an entrance of a fluidic sample. While the opening 951 is designed as an entrance of a fluidic sample, the opening 952 is then designed as a vent, or vice versa. In other words, after a fluidic sample to be tested (e.g., blood) is introduced into the channel 95 via the opening 951, the channel 95 causes a capillary action that draws the fluidic sample to be tested through the channel 95 to the reaction region 950. The reaction region 950 is coated with the reaction materials. Accordingly, the analyte of the fluidic sample to be tested (e.g., blood glucose) would react with the reaction materials to induce an electrochemical reaction. The electrodes 921 then deliver an electrical current signal generated from the electrochemical reaction to an analytical instrument for judging the signal.

Moreover, in order to maintain the capability of the channel 95 to cause a capillary action, the width of the channel 95 is limited so that the size of the opening 951 and the opening 952 would not be too large. In practical use, the user must align the channel 95 and the fluidic sample to be tested (e.g., the blood of a fingertip) precisely so that the fluidic sample to be tested (e.g., the blood of a fingertip) can deliver into the reaction region 950 successfully. However, the users of the electrochemical-based analytical strip 9 include the long-term chronic disease patients (e.g., diabetics) who usually suffer from other complications (such as diabetic maculopathy or peripheral neuropathy) which results in the sight damage or failure of motion coordination. Accordingly, such patients fail to accomplish the above-mentioned operation which requires the precise coordination of the eyes and the hands, resulting in test errors thereby and the frustration to the users.

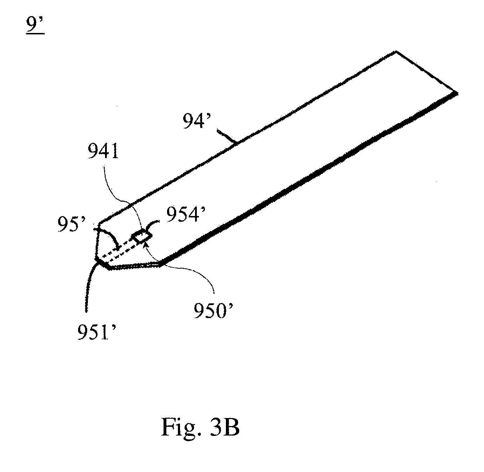

Furthermore, U.S. Pat. No. 6,258,229 discloses another conventional electrochemical-based analytical strip as shown in FIG. 3B. The channel 95' of the conventional electrochemical-based analytical strip 9' extends from its frond end toward its rear end to form a U-shaped structure. The channel 95' which is of the U-shaped structure has an opening 951'. In use, a fluidic sample to be tested is introduced into the channel 95' via the opening 951'. In order to prevent the air bubbles from blocking the channel 95' after the introduction of the fluidic sample to be tested into the channel 95' and to improve the channel 95, as well as to maintain its capability to cause a capillary action and a siphon phenomenon, and thereby to deliver the fluidic sample to be tested into the reaction region 950' successfully, the cover 94' disposes a vent 941' located on the rear end 954' of the channel 95'. Accordingly, the air bubbles tend to be generated in the channel 95' are ejected out from the vent 941'.

In addition, attempts have been made to avoid the above-mentioned drawbacks. For example, WO 2004/113901 A1 discloses a test strip with a slot vent opening used for detecting the fluid sample. US 2006/175199 A1 also discloses an electrochemical sensor strip used for similar purpose, wherein a vent serving as a ventilator is provided for excluding the air. However, conventional electrochemical sensor strips have the disadvantage that it is difficult to insert a sample into a corresponding sample receiving portion and that the provision of the test strip with a vent is complex.

### SUMMARY OF THE INVENTION

Thus, it is the object of the present application to provide a test strip which is configured in a way that a sample can be easily inserted into a reaction chamber and which does not need a vent, thus simplifying the production process.

The above-mentioned object is solved by the testing strip for detecting a fluid sample according to claim 1. Advantageous improvements of the invention are the subject matter of dependent claims.

In particular, in order to overcome the shortcomings of the prior arts mentioned above, the present invention provides a testing strip for detecting a fluidic. The testing strip for detecting a fluidic sample comprises a substrate, a plurality of electrodes, a supporting layer and a cover. The substrate, the electrodes, the supporting layer and the cover are serially stacked. The testing strip for detecting a fluidic sample includes a longitudinal long axis and a transverse short axis. The longitudinal long axis and the transverse short axis are perpendicular to each other. The testing strip for detecting a fluidic sample includes a first end and a second end opposing to the first end along the longitudinal long axis. The testing strip for detecting a fluidic sample includes a reacting region located at the terminal of the first end, and the reacting region is defined and enclosed by the cover, the supporting layer and the substrate. The electrodes extend into the reacting region. The reacting region has a maximum depth along the longitudinal long axis and a maximum width along the transverse short axis, and the maximum width is greater than the maximum depth. The fluidic sample enters into the reacting region and flows within the reacting region along a longest flow-path. The reacting region has a C-shaped structure on a cross-section that is perpendicular to the longest flowing path. The cover has a hydrophilic material coated on a side facing toward the reacting region.

Accordingly, the primary object of the present invention is to provide a testing strip for detecting a fluidic sample whose reacting region is a three-side enclosed area in a cross-sectional view along the longitudinal long axis. When the fluidic sample to be tested enters into the reacting region, the air in the reacting region will be propelled out of the reacting region through the direction other than the entering direction of the fluidic sample to be tested. For this reason, during the manufacturing process, the cover, the supporting layer, or the electrodes is not necessarily disposed any vent. In addition, it is not necessary to align the cover and the channel (the reacting region) precisely, thereby to reduce the manufacturing costs and to improve the manufacturing yield.

Another object of the present invention is to provide to provide a testing strip for detecting a fluidic sample whose reacting region is disposed at the terminal of the first end, and the maximum width of the reacting region is greater than the maximum depth of the reacting region. As a result, the fluidic sample to be tested can be introduced into the reaction region from anywhere of the reaction region that is open. After the introduction of the fluidic sample to be tested into the reacting region, the air in the reacting region are propelled out of the reacting region through the direction other than the entering direction for the fluidic sample to be tested. Hence, the users do not need to precisely align the testing strip for detecting a fluidic sample and the sampled region of the users. Consequently, it is convenient for long-term chronic disease patients and elders to employ the testing strip for detecting a fluidic sample of the present invention.

Yet another object of the present invention is to provide a testing strip for detecting a fluidic sample that has a pair of bevel edges respectively disposed at each lateral side of the first end with respect to the longitudinal long axis of the testing strip for detecting a fluidic sample. The user can simply apply the guiding structure of the testing strip for detecting a fluidic sample to the sampled region of the user, thereby resulting in the fluidic sample to be tested being drawn into the reacting region by a capillary force and therefore to facilitate the sampling of the fluidic sample. Accordingly, the air is propelled out of the reacting region in the direction toward the first end and the direction toward the other guiding structure.

### BRIEF DESCRIPTION OF THE DRAWINGS

The invention as well as a preferred mode of use, further objectives and advantages thereof, will best be understood by reference to the following detailed description of an illustrative embodiment when read in conjunction with the accompanying drawings, wherein:
FIG. 1A is a perspective assembly view of a testing strip for detecting a fluidic sample according to the first preferred embodiment of the present invention;
FIG. 1B is a perspective disassembly view of a testing strip for detecting a fluidic sample according to the first preferred embodiment of the present invention;
FIG. 1C is an enlarged view of the first end of a testing strip for detecting a fluidic sample according to the first preferred embodiment of the present invention;
FIG. 1D is a top view of a testing strip for detecting a fluidic sample according to the first preferred embodiment of the present invention;
FIG. 1E is a top view of a testing strip for detecting a fluidic sample according to the first preferred embodiment of the present invention;
FIG. IF is a cross-sectional view of a testing strip for detecting a fluidic sample taken along the line AA of FIG. 1A according to the first preferred embodiment of the present invention;
FIG. 2A is a perspective assembly view of a testing strip for detecting a fluidic sample according to the second preferred embodiment of the present invention;
FIG. 2B is a perspective disassembly view of a testing strip for detecting a fluidic sample according to the second preferred embodiment of the present invention;
FIG. 2C is an enlarged view of the first end of a testing strip for detecting a fluidic sample according to the second preferred embodiment of the present invention;
FIG. 2D is a cross-sectional view of a testing strip for detecting a fluidic sample taken along the line AA of FIG. 2A according to the second preferred embodiment of the present invention;
FIG. 3A is a perspective view of a conventional electrochemical-based analytical strip; and
FIG. 3B is a perspective view of a conventional electrochemical-based analytical strip disclosed in U.S. Pat. No. 6,258,229.

### DETAILED DESCRIPTION OF THE PREFERRED EMBODIMENT

The scope of protection is defined by the claims.

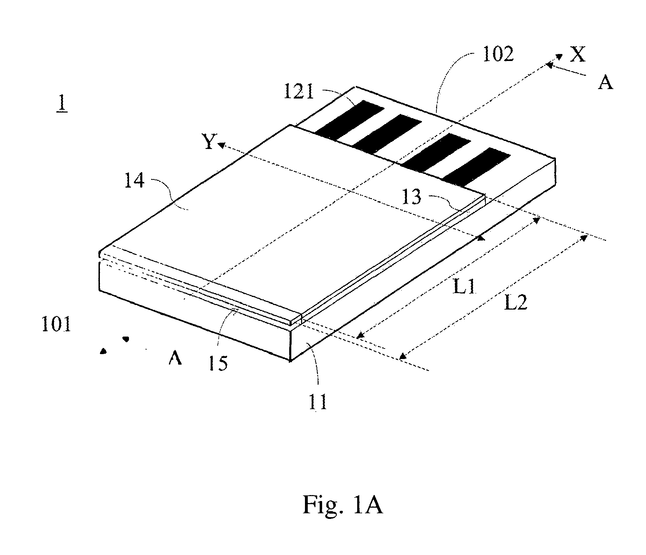

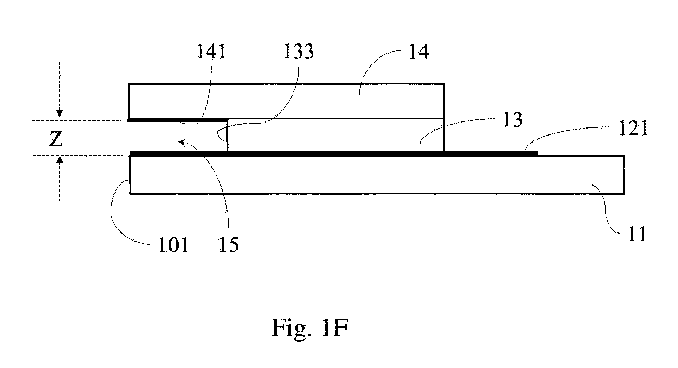

Please refer to FIG. 1A, which is a perspective assembly view of a testing strip for detecting a fluidic sample according to the first preferred embodiment of the present invention. The testing strip for detecting a fluidic sample 1 comprises a substrate 11, a plurality of electrodes 121, a supporting layer 13 and a cover 14. In particular, the substrate 1 is preferably made of a bio-inert material.

The testing strip for detecting a fluidic sample 1 includes a longitudinal long axis X and a transverse short axis Y. The longitudinal long axis X and the transverse short axis Y are perpendicular to each other. The testing strip for detecting a fluidic sample 1 includes a first end 101 and a second end 102 and the second end 102 is opposite to the first end 101 along the longitudinal long axis X.

Along the longitudinal long axis X, the length L2 of the supporting layer 13 is smaller than the length L1 of the cover 14. The cover 14 is mounted on the supporting layer 13. The testing strip for detecting a fluidic sample 1 includes a reacting region 15 located at the terminal of the first end 101, and the reacting region 15 is defined and enclosed by the cover 14, the supporting layer 13 and the substrate 11. Particularly, the cover 14 completely covers on the reacting region 15. In addition, the reacting region 15 is parallel to the transverse short axis Y. In practical use, the user can simply apply the first end 101 of the testing strip for detecting a fluidic sample 1 to the sampled region, for example, the skin at an acupuncture point, thereby resulting in a fluidic sample to be tested being drawn into the reacting region 15 by a capillary force and therefore to facilitate the sampling of the fluidic sample.

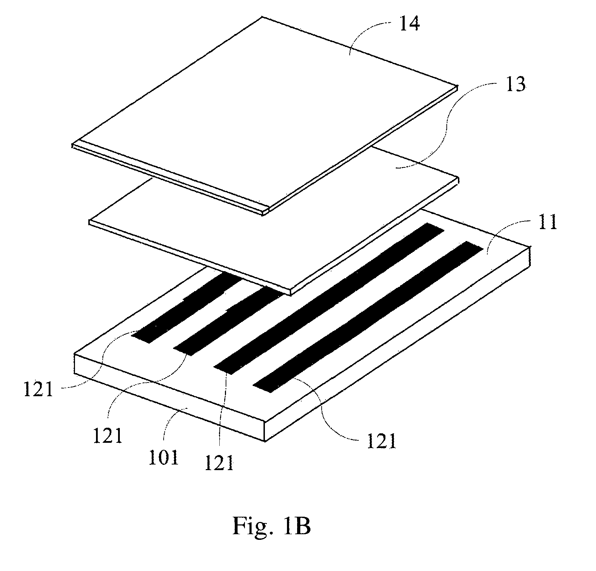

Please refer to FIG. 1B, which is a perspective disassembly view of a testing strip for detecting a fluidic sample 1 according to the first preferred embodiment of the present invention. A substrate 11, a plurality of electrodes 121, a supporting layer 13 and a cover 14 are serially stacked from bottom to top. The electrodes 121 are mounted on the substrate 11 by means of printing, coating or deposition.

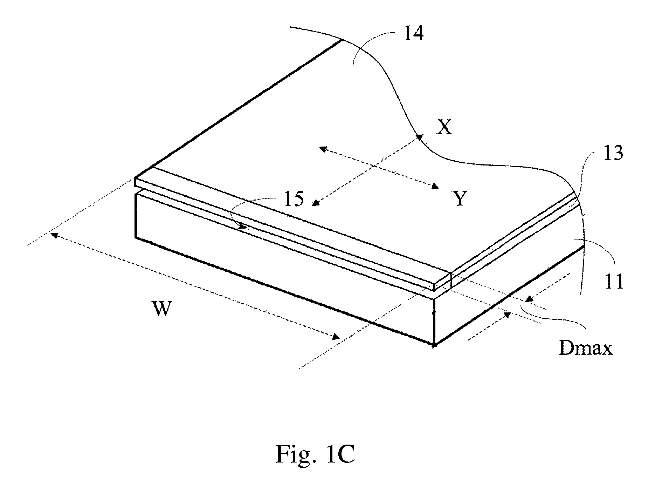

Please refer to FIG. 1C, which is an enlarged view of the first end of the testing strip for detecting a fluidic sample 1 according to the first preferred embodiment of the present invention. The reacting region 15 has a maximum depth Dmax along the longitudinal long axis X and a maximum width W along the transverse short axis Y, and the maximum width W is greater than the maximum depth Dmax. The reacting region 15 preferably has a volume that is not greater than 5 microliters, and more preferably, the reacting region 15 has a volume of 1 microliter.

Moreover, as described above, the maximum width W is greater than the maximum depth Dmax. The maximum depth Dmax and the maximum width W preferably have a ratio that is not greater than 1:2, and more preferably, the maximum depth Dmax and the maximum width W have a ratio of 1:5.

The purpose of using the above-mentioned design is to facilitate a fluidic sample to be tested to enter into the reacting region 15 from any direction by a capillary force. Thereby, the fluid sample fills the whole reacting region 15. And, the air in the reacting region 15 will be propelled by the fluidic sample to be tested out of the reacting region 15 through the direction other than the entering direction for the fluidic sample to be tested.

For this reason, the cover 14 of the present invention does not need to dispose any vent. Thereby, the manufacturing costs are reduced and the manufacturing yield is improved. In addition, the testing strip for detecting a fluidic sample 1 includes an entrance (the reacting region 15) for the fluidic sample to be tested located at the terminal side of its front end (the first end 101), and the reacting region 15 has a maximum depth Dmax along the longitudinal long axis X and a maximum width W along the transverse short axis Y, wherein the maximum width W is greater than the maximum depth Dmax. As a result, any open place of the reacting region 15 can receive the induction of the fluidic sample to be tested. Even though, for example, when the fluidic sample to be tested is introduced into the central portion of the reacting region 15, the air in the reacting region 15 can still be pushed and propelled by the fluidic sample out from the center to the both sides of the reacting region 15. Hence, the users do not need to precisely align the testing strip 1 and the sampled region of the user. Consequently, it is convenient for long-term chronic disease patients and elders to employ the testing strip for detecting a fluidic sample 1 of the present invention.

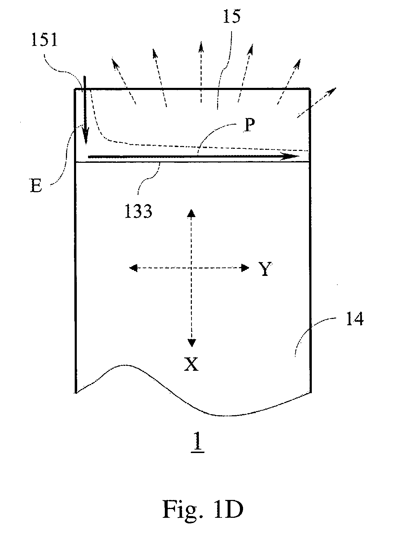

For example, please refer to FIG. 1D, which is a top view of a testing strip for detecting a fluidic sample according to the first preferred embodiment of the present invention. When the user makes the fluidic sample contact with the edge 151 of the reacting region 15 of the testing strip for detecting a fluidic sample 1, the fluidic sample is introduced into the reacting region 15 along the entering direction E which is parallel to the longitudinal long axis X. Through a shortest path (the above-mentioned entering direction E), the fluidic sample is drawn into and filled the reacting region 15 by a capillary force. Later, the fluidic sample contacts with the side 133 of the supporting layer 13 that faces toward the first end 101, and then the fluidic sample flows along the flow-path P which is parallel to the transverse short axis Y due to that the interaction between the cohesive force of the fluidic sample itself and the adhesive force formed between the fluidic sample and the said side 133. Specifically, since the maximum width W of the reacting region 15 (i.e., the longest flowing distance of the fluidic sample along the flow-path P) along the transverse short axis Y is greater than the maximum depth Dmax (i.e., the longest flowing distance of the fluidic sample along the entering direction E) along the longitudinal long axis X, in the reaction region 15, the longest flow-path of the fluidic sample is the flow-path P. Therefore, when the fluidic sample flows along the entering direction E and the flow-path P, the fluidic sample delineates an interface like the dotted curved line shown in the Fig.1D. At the same time, the air in the reacting region 15 is pushed and propelled out by the fluidic sample from the reacting region 15 through the direction (i.e., the dotted arrow in the Fig.1D) other than the entering direction E for the fluidic sample.

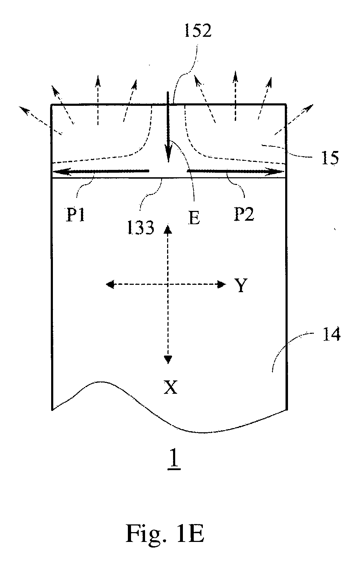

Furthermore, please refer to FIG. 1E, which is a top view of a testing strip for detecting a fluidic sample according to the first preferred embodiment of the present invention. When the user makes the fluidic sample contact with the middle portion 152 (but not necessary the precise central portion) of the reacting region 15 of the testing strip for detecting a fluidic sample 1, similarly as mentioned above, the fluidic sample is introduced into the reacting region 15 along the entering direction E which is parallel to the longitudinal long axis X. The fluidic sample is then drawn into the reacting region 15 by a capillary force through a shortest path (i.e., the entering direction E). Later, when the fluidic sample contacts with the side 133 of the supporting layer 13 that faces toward the first end 101, the fluidic sample respectively flows along the path P1 and path P2 (both are parallel to the transverse short axis Y) due to the interaction between the cohesive force of the fluidic sample itself and the adhesive force formed between the fluidic sample and the said side 133. Under such circumstance, in the reaction region 15, the fluidic sample has a longest flow-path that equals to the sum of the path P1 and path P2. Likewise, either when the fluidic sample flows along the entering direction E and then the path P1, or when it flows along the entering direction E and then the path P2, the fluidic sample delineates an interface like the dotted curved line shown in the Fig.1E. At the same time, the air in the reacting region 15 are pushed and propelled by the fluidic sample out from the reacting region 15 through the direction (i.e., the dotted arrow shown in the Fig.1E) other than the entering direction E for the fluidic sample.

Please refer to FIG. IF, which is a cross-sectional view of a testing strip for detecting a fluidic sample taken along the line AA of FIG. 1A according to the first preferred embodiment of the present invention. Specifically, the line AA is perpendicular to the above-mentioned flow-path P (or the sum of path P1 and path P2) of the fluidic sample. The cover 14 is mounted on the supporting layer 13, and the cover 14 completely covers on the reacting region 15. As a result, the cover 14 has a cantilever structure formed at the first end 101 of the testing strip for detecting a fluidic sample 1 (as shown in FIG. 1A). The electrodes 121 extend into the reacting region 15. The reacting region 15 is defined and enclosed by the cover 14, supporting layer 13 and the substrate 11. In addition, as shown in FIG. IF, the reacting region 15 has a C-liked structure from the cross-sectional view of the test strip 1 along the AA line. Moreover, if the surface tension force of the fluidic sample is greater than the capillary force formed between the fluidic sample and the reacting region 15, the introduction of the fluidic sample to be tested into the reacting region 15 becomes slow. Accordingly, in order to keep the reacting region 15 having desirable capillary force, the height Z and the maximum depth Dmax (as shown in FIG. 1C) of the reacting region 15 are preferable in a ratio of at least 1:20, and more preferable in a ratio of at least 1:10. In other words, while the height Z of the reacting region 15 is 100 µm (micrometer), the maximum depth Dmax of the reacting region 15 along the longitudinal long axis X is preferably 1 mm (millimeter).

Furthermore, the cover 14 has a hydrophilic material coated on a side facing toward the reacting region 15, thereby making the fluidic sample being more smoothly and easily delivered into the reacting region 15. In addition, if the fluidic sample to be tested is insufficient to fill the whole reacting region 15, this will lead to erroneous test results. To prevent this from happening, an end of the cover 14 that is close to the first end 101 is preferably made of a transparent material so that the user can easily observe the condition of the delivery of the fluidic sample through the reacting region 15.

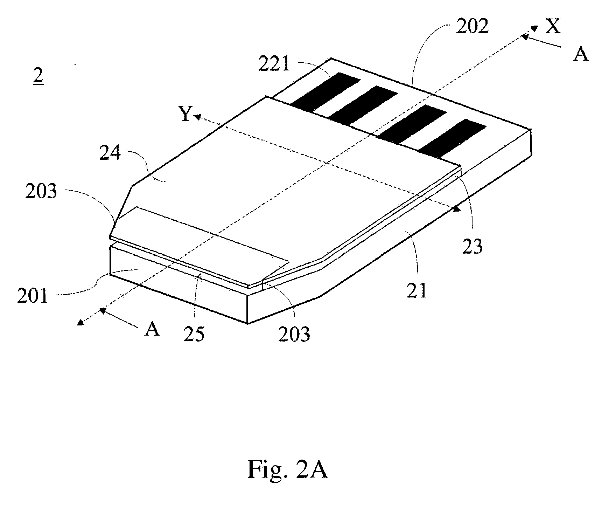

In addition, please refer to FIG. 2A through FIG. 2D, which depict another testing strip for detecting a fluidic sample according to the second preferred embodiment of the present invention. It is to be noted that the testing strip for detecting a fluidic sample mentioned in the second preferred embodiment possess the features which are substantially the same as those of the first preferred embodiment. The differences between the testing strip for detecting a fluidic sample of the second preferred embodiment and the first preferred embodiment are recited below.

Referring to FIG. 2A, the testing strip for detecting a fluidic sample 2 includes a longitudinal long axis X and a transverse short axis Y, and the longitudinal long axis X and the transverse short axis Y are perpendicular to each other. The testing strip for detecting a fluidic sample 2 includes a first end 201 and a second end 202 opposing to the first end 201 along the longitudinal long axis X. A pair of bevel edges 203 are respectively disposed at each lateral sides of the first end 201 with respect to the longitudinal long axis X of the testing strip for detecting a fluidic sample 2. Each of bevel edges 203 inclined from the first end 201 toward the second end 202. In addition, as shown in FIG. 2A, each of the bevel edges 203 is of straight-line shape; however, with a particular requirement, each of the bevel edges 203 can also be of arc or rounded shape. Consequently, in practical use, the user can simply apply the bevel edges 203 of the testing strip for detecting a fluidic sample 2 at the sampled region, for example, the skin at an acupuncture point, thereby resulting in drawing a fluidic sample to be tested into the reacting region 25 through the capillary force to facilitate the sampling of the fluidic sample.

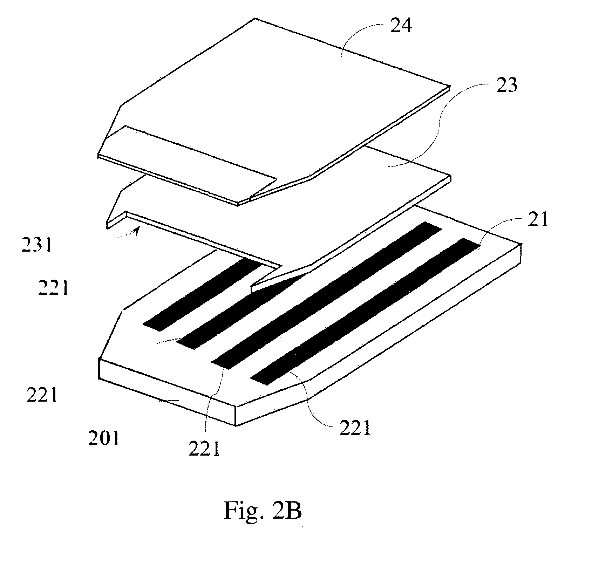

Please refer to FIG. 2B, which is a perspective disassembly view of a testing strip for detecting a fluidic sample according to the second preferred embodiment of the present invention. A substrate 21, a plurality of electrodes 221, a supporting layer 23 and a cover 24 are serially stacked from bottom to top. The supporting layer 23 has a recess 231 formed at the first end 201. The electrodes 221 extend into the reacting region 25 (as shown in FIG. 2A).

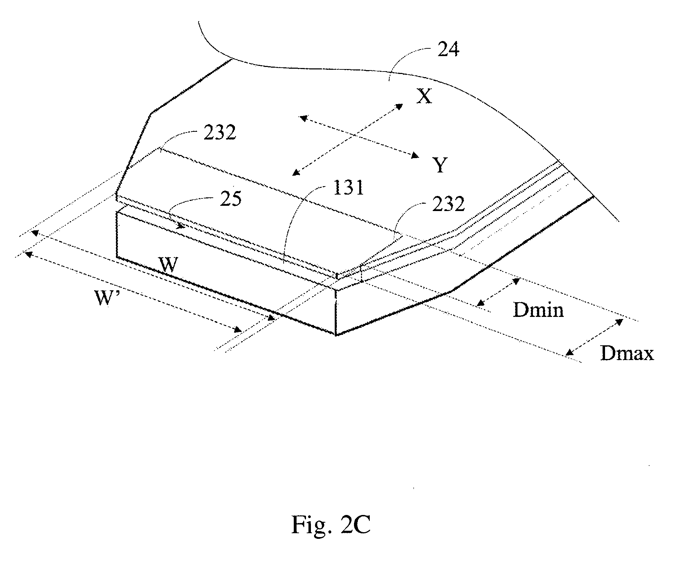

Referring to FIG. 2C, which is an enlarged view of the first end of the testing strip for detecting a fluidic sample 2 according to the second preferred embodiment of the present invention. Since a pair of guiding structures 203 is respectively disposed at each lateral sides of the first end 201, the recess 231 is a trapezoid-like structure, rather than a perfect rectangular structure. Therefore, the recess 231 further has a minimum depth Dmin along the longitudinal long axis X. Moreover, the minimum depth Dmin of the recess 231 and the maximum depth Dmax of the reacting region 25 are preferable in a ratio that is not greater than 3:5, and more preferable in a ratio that is not greater than 2:5. In addition, in the recess 231 and near the second end 202, a pair of rounded edges 232 is respectively disposed on each of the two lateral sides of the recess 231 with respect to the longitudinal long axis X (as shown in FIG. 2A). The recess 231 is designed as above-mentioned for that when the fluidic sample to be tested flows into the recess 231, vortex flow or turbulent flow can be reduced, thereby preventing the generation of the air bubbles. Thus, an accurate result of the quantitative assay could be assured. Each of the rounded edges 232 that is disposed near the second end 202 has a radius of curvature (R) of preferable at least 0.5 millimeter.

Moreover, as described above, the maximum width W of the reacting region 25 is greater than the maximum depth Dmax of the reacting region 25. Therefore, the maximum depth Dmax and the maximum width W preferably are preferable in a ratio that is not greater than 1:2, and more preferably, the maximum depth Dmax and the maximum width W are in a ratio of 1:5.

The purpose of using the above-mentioned design is to facilitate a fluidic sample to be tested, from any direction, enter into the reacting region 25 from either side of the recess 231 near the pair of bevel edges 203 by a capillary force. Accordingly, the air in the reacting region 25 are pushed and propelled by the fluidic sample to be tested toward the first end 201 and the other bevel edge 203, then out of the reacting region 25. Therefore, the cover 24 of the present invention does not need to dispose any vent. During the manufacturing process, the cover 24 does not have to be necessarily aligned with the recess 231 precisely. Thereby, the manufacturing costs are reduced and the manufacturing yield is improved. In addition, the testing strip for detecting a fluidic sample 2 has an entrance (the reacting region 25) for the fluidic sample to be tested located at the terminal of its front end (the first end 201). And, the reacting region 25 has a maximum depth Dmax along the longitudinal long axis X and a maximum width W and a maximum opening width W' along the transverse short axis Y. Both of the maximum width W and the maximum opening width W' are greater than the maximum depth Dmax. As a result, when the fluidic sample to be tested is introduced, the air in the reacting region 25 can be pushed and propelled by the fluidic sample to be tested, from the center of the reacting region 25 to the bevel edges 203. Hence, the users do not necessarily align the testing strip for detecting a fluidic sample 2 and the sampled region precisely. Consequently, it is convenient for long-term chronic disease patients and elders to employ the testing strip for detecting a fluidic sample 2 of the present invention.

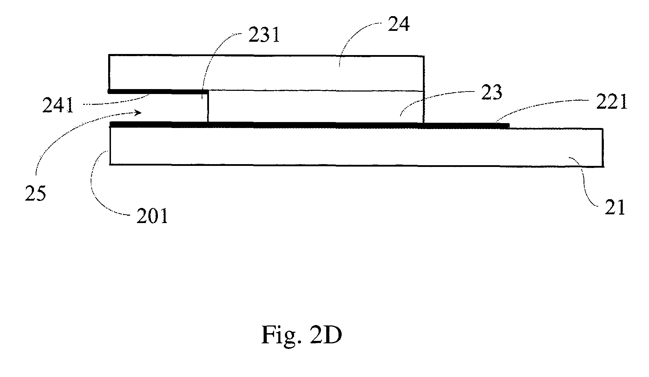

Please refer to FIG. 2D, which is a cross-sectional view of a testing strip for detecting a fluidic sample 2 taken along the line AA of FIG. 2A according to the second preferred embodiment of the present invention. The cover 24 is mounted on the supporting layer 23, and the cover 24 completely covers on the reacting region 25. The reacting region 25 is defined and enclosed by the cover 24, the supporting layer 23 and the substrate 21. Furthermore, the cover 24 has a hydrophilic material coated on a side facing toward the reacting region 25 to make the fluidic sample to be more easily introduced into the reacting region 25 from the recess 231. In addition, if the fluidic sample to be tested is insufficient to fill the whole reacting region 25, it will lead to errors of test results. To prevent this from happening, an end of the cover 24 that is close to the first end 201 is preferably made of a transparent material so that the user can easily observe the condition of the delivery of the fluidic sample through the reacting region 25. Moreover, the height Z of the reacting region 25 and the maximum depth Dmax of the reacting region 25 along the longitudinal long axis X are in a ration that is substantially the same as that of the first preferred embodiment and therefore omitted herein.

## Claims

1. A testing strip for detecting a fluidic sample (1, 2) for testing a fluidic sample, comprising a substrate (11, 21), a plurality of electrodes (121, 221), a supporting layer (13, 23) and a cover (14, 24), which are stacked serially, wherein the testing strip for detecting a fluidic sample (1, 2) includes a longitudinal axis (X) and a transverse axis (Y), the longitudinal axis (X) being perpendicular to the transverse axis (Y), the testing strip for detecting a fluidic sample (1, 2) including a first end (101, 201) and a second end (102, 202) opposing to the first end (101, 201) along the longitudinal axis (X), the testing strip for detecting a fluidic sample (1, 2) being **characterized in that**:
the testing strip for detecting a fluidic sample (1, 2) includes a reacting region (15, 25) located at a terminal of the first end (101, 201), wherein the reacting region (15, 25) is parallel to the transverse axis (Y) and is defined and enclosed by the cover (14, 24), supporting layer (13, 23) and the substrate (11, 21), and the electrodes (121, 221) extend into the reacting region (15, 25);
the reacting region (15, 25) has a maximum depth (Dmax) along the longitudinal axis (X) and a maximum width (W) along the transverse axis (Y), wherein the maximum depth (Dmax) and the maximum width (W) have a ratio that is not greater than 1:2;
when the fluidic sample enters into the reacting region (15, 25) and flows within the reacting region (15, 25), the fluidic sample has a longest flow-path (P, P1, P2) within the reacting region (15, 25), wherein the reacting region (15, 25) has a C-liked structure from a cross-sectional view taken along a direction perpendicular to the longest flowing path (P, P1, P2); and
the cover (14, 24) has a hydrophilic material coated on a side facing toward the reacting region (15, 25).

2. The testing strip for detecting a fluidic sample (1, 2) of claim 1, wherein a pair of bevel edges (203) are respectively disposed at each lateral sides of the first end (201) with respect to the longitudinal axis (X) of the testing strip for detecting a fluidic sample (2), each of the bevel edges (203) inclining from the first end (201) toward the second end (202).

3. The testing strip for detecting a fluidic sample (2) of claim 2, wherein the supporting layer (23) has a recess (231) formed at the first end (201), and the recess (231) further has a minimum depth (Dmin) along the longitudinal axis (X), and the minimum depth (Dmin) of the recess (231) and the maximum depth (Dmax) of the reacting region (25) are in a ratio that is not greater than 3:5.

4. The testing strip for detecting a fluidic sample (2) of claim 3, wherein the minimum depth (Dmin) and the maximum depth (Dmax) are preferably in a ratio that is not greater than 1:5.

5. The testing strip for detecting a fluidic sample (2) of claim 3, wherein a pair of rounded edges (232) are respectively disposed in the recess (231) near each corner of the recess (231) at two lateral sides with respect to the longitudinal axis (X), and each of the rounded edges (232) has a radius of curvature (R) of at least 0.5 millimeter.

6. The testing strip for detecting a fluidic sample (2) of claim 2, wherein each of the bevel edges (203) is of arc shape or straight-line shape.

7. The testing strip for detecting a fluidic sample (1, 2) of claim 1, wherein the reacting region (15, 25) has a volume of at most 5 microliters.

8. The testing strip for detecting a fluidic sample (1, 2) of claim 1, wherein the substrate (11, 21) is made of a bio-inert material.

9. The testing strip for detecting a fluidic sample (1, 2) of claim 1, wherein an end of the cover (14, 24) that is close to the first end (101, 201) is made of a transparent material.

## Patentansprüche

1. Teststreifen zum Detektieren einer Flüssigkeitsprobe (1,2) zum Testen einer Flüssigkeitsprobe, der ein Substrat (11,21), eine Vielzahl von Elektroden (121, 221) eine Stützschicht (13, 23) und eine Abdeckung (14, 24) aufweist, welche in Reihenfolge gestapelt sind, wobei der Teststreifen zum Detektieren einer Flüssigkeitsprobe (1, 2) eine Längsachse (X) und eine Querachse (Y) aufweist, wobei die Längsachse (X) rechtwinklig zu der Querachse (Y) ist, wobei der Teststreifen zum Detektieren einer Flüssigkeitsprobe (1, 2) entlang der Längsachse (X) ein erstes Ende (101, 201) und ein zweites Ende (102, 202) gegenüber von dem ersten Ende (101, 201) aufweist, wobei der Teststreifen zum Detektieren einer Flüssigkeitsprobe (1,2) **dadurch gekennzeichnet ist, dass**:
der Teststreifen zum Detektieren einer Flüssigkeitsprobe (1, 2) einen Reaktionsbereich (15, 25) aufweist, der an einem Ende des ersten Endes (101, 201) angeordnet ist, wobei der Reaktionsbereich (15, 25) parallel zu der Querachse (Y) ist und durch die Abdeckung (14,24), die Stützschicht (13, 23) und das Substrat (11, 21) definiert und umschlossen ist, wobei sich die Elektroden (121, 221) in den Reaktionsbereich (15, 25) hinein erstrecken;
der Reaktionsbereich (15, 25) entlang der Längsachse (X) eine maximale Tiefe (Dmax) und entlang der Querachse (Y) eine maximale Breite (W) aufweist, wobei die maximale Tiefe (Dmax) und die maximale Breite (W) ein Verhältnis aufweisen, das nicht größer ist als 1:2;
wobei, wenn die Flüssigkeitsprobe in den Reaktionsbereich (15, 25) eintritt und innerhalb des Reaktionsbereichs (15, 25) fließt, die Flüssigkeitsprobe einen längsten Flussweg (P, P1, P2) innerhalb des Reaktionsbereichs (15, 25) aufweist, wobei der Reaktionsbereich (15, 25) betrachtet in einer Querschnittsansicht entlang einer Richtung rechtwinklig zu dem längsten Flussweg (P, P1, P2) eine C-ähnliche Struktur aufweist; und
die Abdeckung (14, 24) ein hydrophiles Material aufweist, das an einer Seite, die auf den Reaktionsbereich (15, 25) zuweist, aufgetragen ist.

2. Teststreifen zum Detektieren einer Flüssigkeitsprobe (1, 2) nach Anspruch 1, wobei ein Paar von schrägen Kanten (203) jeweils an jeder lateralen Seite des ersten Endes (201) in Bezug auf die Längsachse (X) des Teststreifens zum Detektieren einer Flüssigkeitsprobe (2) angeordnet ist, wobei jede der schrägen Kanten (203) von dem ersten Ende (201) hin zu dem zweiten Ende (202) geneigt ist.

3. Teststreifen zum Detektieren einer Flüssigkeitsprobe (2) nach Anspruch 2, wobei die Stützschicht (23) eine Ausnehmung (231) aufweist, die an dem ersten Ende (201) ausgebildet ist, wobei die Ausnehmung (231) ferner entlang der Längsachse (X) eine minimale Tiefe (Dmin) aufweist, wobei die minimale Tiefe (Dmin) der Ausnehmung (231) und die maximale Tiefe (Dmax) des Reaktionsbereichs (21) ein Verhältnis aufweisen, das nicht größer als 3:5 ist.

4. Teststreifen zum Detektieren einer Flüssigkeitsprobe (2) nach Anspruch 3, wobei die minimale Tiefe (Dmin) und die maximale Tiefe (Dmax) ein Verhältnis aufweisen, das nicht größer als 1:5 ist.

5. Teststreifen zum Detektieren einer Flüssigkeitsprobe (2) nach Anspruch 3, wobei ein Paar von runden Kanten (232) jeweils in der Ausnehmung (231) nahe jeder Ecke der Ausnehmung (231) an zwei lateralen Seiten in Bezug auf die Längsachse (X) angeordnet sind und wobei jede der runden Kanten (232) einen Krümmungsradius (X) von mindestens 0,5 Millimeter aufweist.

6. Teststreifen zum Detektieren einer Flüssigkeitsprobe (2) nach Anspruch 2, wobei jede der schrägen Kanten (203) eine Bogenform oder eine geradlinige Form aufweist.

7. Teststreifen zum Detektieren einer Flüssigkeitsprobe (1, 2) nach Anspruch 1, wobei der Reaktionsbereich (15, 25) ein Volumen von höchstens 5 Mikrolitern aufweist.

8. Teststreifen zum Detektieren einer Flüssigkeitsprobe (1, 2) nach Anspruch 1, wobei das Substrat (11, 21) aus einem bio-inerten Material hergestellt ist.

9. Teststreifen zum Detektieren einer Flüssigkeitsprobe (1, 2) nach Anspruch 1, wobei ein Ende der Abdeckung (14,24), das näher zu dem ersten Ende (101,201) ist, aus einem transparenten Material hergestellt ist.

## Revendications

1. Bandelette d'essai pour détecter un échantillon fluidique (1, 2) pour tester un échantillon comprenant un substrat (11, 21), une pluralité d'électrodes (121, 221), une couche de support (13, 23) et un couvercle (14, 24) qui sont empilés en série, dans laquelle la bandelette d'essai pour détecter un échantillon fluidique (1, 2) inclut un axe longitudinal (X) et un axe transversal (Y), l'axe longitudinal (X) étant perpendiculaire à l'axe transversal (Y), la bandelette d'essai pour détecter un échantillon fluidique (1, 2) incluant une première extrémité (101, 201) et une seconde extrémité (102, 202) opposée à la première extrémité (101, 201) le long de l'axe longitudinal (X), la bandelette d'essai pour détecter un échantillon fluidique (1, 2) étant **caractérisée en ce que** :
la bandelette d'essai pour détecter un échantillon fluidique (1, 2) inclut une région réactive (15, 25) située à un terminus de la première extrémité (101, 201), la région réactive (15, 25) étant parallèle à l'axe transversal (Y) et étant définie et enfermée par le couvercle (14, 24), la couche de support (13, 23) et le substrat (11, 21) et les électrodes (121, 221) s'étendant dans la région réactive (15, 25) ;
la région réactive (15, 25) a une profondeur maximale (Dmax) le long de l'axe longitudinal (X) et une largeur maximale (W) le long de l'axe transversal (Y), la profondeur maximale (Dmax) et la largeur maximale (W) ayant un rapport qui n'est pas supérieur à 1:2,
lorsque l'échantillon fluidique entre dans la région réactive (15, 25) et s'écoule à l'intérieur de la région réactive (15, 25), l'échantillon fluidique a une voie d'écoulement la plus longue (P, P1, P2) à l'intérieur de la région réactive (15, 25), la région réactive (15, 25) ayant une structure semblable à un C à partir d'une vue en section transversale le long d'une direction perpendiculaire à la voie d'écoulement la plus longue (P, P1, P2) et
le couvercle (14, 24) a un matériau hydrophile revêtu sur un côté tourné vers la région réactive (15, 25).

2. Bandelette d'essai pour détecter un échantillon fluidique (1, 2) selon la revendication 1 dans laquelle une paire de bords biseautés (203) est disposée respectivement sur chaque côté latéral de la première extrémité (201) par rapport à l'axe longitudinal (X) de la bandelette d'essai pour détecter un échantillon fluidique, chacun des bords biseautés (203) étant incliné de la première extrémité (201) vers la seconde extrémité (202).

3. Bandelette d'essai pour détecter un échantillon fluidique (2) selon la revendication 2 dans laquelle la couche de support (23) a un évidement (231) formé à la première extrémité (201) et l'évidement (231) a de plus une profondeur minimale (Dmin) le long de l'axe longitudinal (X) et la profondeur minimale (Dmin) de l'évidement (231) et la profondeur maximale (Dmax) de la région réactive (25) sont dans un rapport qui n'est pas supérieur à 3:5.

4. Bandelette d'essai pour détecter un échantillon fluidique (2) selon la revendication 3 dans laquelle la profondeur minimale (Dmin) et la profondeur maximale (Dmax) sont de préférence dans un rapport qui n'est pas supérieur à 1:5.

5. Bandelette d'essai pour détecter un échantillon fluidique (2) selon la revendication 3 dans laquelle une paire de bords arrondis (232) est disposée respectivement dans l'évidement (231) près de chaque coin de l'évidement (231) sur deux côtés latéraux par rapport à l'axe longitudinal (X) et chacun des bords arrondis (232) a un rayon de courbure (R) d'au moins 0,5 millimètre.

6. Bandelette d'essai pour détecter un échantillon fluidique (2) selon la revendication 2 dans laquelle chacun des bords biseautés (203) est de forme arquée ou de forme en ligne droite.

7. Bandelette d'essai pour détecter un échantillon fluidique (1, 2) selon la revendication 1 dans laquelle la région réactive (15, 25) a un volume d'au maximum 5 microlitres.

8. Bandelette d'essai pour détecter un échantillon fluidique (1, 2) selon la revendication 1 dans laquelle le substrat (11, 21) est en matériau bio-inerte.

9. Bandelette d'essai pour détecter un échantillon fluidique (1, 2) selon la revendication 1 dans laquelle une extrémité du couvercle (14, 24) qui est proche de la première extrémité (101, 201) est en matériau transparent.
